# EUROPEAN PATENT APPLICATION

(11) **EP 3 770 913 A1**
(43) Date of publication of application: **27.01.2021**
(21) Application number: 19771206.0
(22) Date of filing: 01.03.2019
(51) Int. Cl.: G16H 30/40, A61B 1/00, A61B 1/045, A61B 34/20, G06Q 10/04

(54) **OPERATION ASSISTANCE SYSTEM, INFORMATION PROCESSING DEVICE, AND PROGRAM**

(30) Priority: 20.03.2018 JP 2018052388
(71) Applicant: Sony Corporation, 108-0075 Tokyo (JP)
(72) Inventor: KONNO, Georgero, Tokyo 108-0075 (JP); NAKAMURA, Naoto, Tokyo 108-0075 (JP); UCHIDA, Masaki, Tokyo 108-0075 (JP); ITO, Toshiki, Tokyo 108-0075 (JP)
(74) Representative: D Young & Co LLP
(86) International application number: PCT/JP2019/008027
(87) International publication number: WO 2019/181432

(57) **Abstract**

[Overview]

[Problem to be Solved] To provide a surgery support system, an information processing apparatus, and a program.

[Solution] A surgery support system including: a storage that stores a determiner, the determiner being obtained by learning a surgical image group using, as supervised data, label information indicating a dangerous condition during surgery; and a predictor that performs a prediction of occurrence of a dangerous condition by using a surgical image as an input and using the determiner.

## Description

### Technical Field

The present disclosure relates to a surgery support system, an information processing apparatus, and a program.

### Background Art

In an operating room, various cameras such as an endoscopic camera, a surgical field camera, and an operating room camera are used, and surgical images obtained by imaging performed by those cameras are displayed during surgery and may be recorded. The recorded surgical images are used to verify and confirm postoperative techniques for a purpose of improving techniques, and are used by doctors in presentations and lectures at academic conferences, for example.

In addition, PTL 1 discloses a technique of automatically adding metadata to a surgical image at a time of imaging during surgery to cause an efficiency of later editing of the recorded surgical image to be improved.

### Citation List

### Patent Literature

PTL 1: Japanese Unexamined Patent Application Publication No. 2016-42982

### Summary of the Invention

### Problems to be Solved by the Invention

However, such a surgical image has not been fully utilized at present, and further utilization of the surgical image has been desired.

### Means for Solving the Problems

According to the present disclosure, there is provided a surgery support system including: a storage that stores a determiner, the determiner being obtained by learning a surgical image group using, as supervised data, label information indicating a dangerous condition during surgery; and a predictor that performs a prediction of occurrence of a dangerous condition by using a surgical image as an input and using the determiner.

Further, according to the present disclosure, there is provided an information processing apparatus including: a storage that stores a determiner, the determiner being obtained by learning a surgical image group using, as supervised data, label information indicating a dangerous condition during surgery; and a predictor that performs a prediction of occurrence of a dangerous condition by using a surgical image as an input and using the determiner.

Further, according to the present disclosure, there is provided a program causing a computer to implement a function of storing a determiner, the determiner being obtained by learning a surgical image group using, as supervised data, label information indicating a dangerous condition during surgery, and a function of performing a prediction of occurrence of a dangerous condition by using a surgical image as an input and using the determiner.

### Effects of the Invention

As described above, according to the present disclosure, it is possible to predict the occurrence of the dangerous condition by utilizing the surgical image.

It is to be noted that the above-described effect is not necessarily limitative and any effect described herein or any other effect understandable herefrom may be achieved in addition to or instead of the above-described effect.

### Brief Description of Drawings

[FIG. 1] FIG. 1 is a block diagram illustrating a schematic configuration of a surgery support system 1000 according to an embodiment of the present disclosure.
[FIG. 2] FIG. 2 illustrates an example of an image including an alert that is displayed in a case where occurrence of a dangerous condition is predicted.
[FIG. 3] FIG. 3 is a diagram illustrating a display example of a manipulation screen.
[FIG. 4] FIG. 4 is a block diagram illustrating a configuration example of a server 10 according to the embodiment.
[FIG. 5] FIG. 5 is a flowchart illustrating an example of an operation related to learning.
[FIG. 6] FIG. 6 is a flowchart illustrating an example of an operation related to a prediction of a dangerous condition.
[FIG. 7] FIG. 7 is an explanatory diagram illustrating a hardware configuration example.

### Modes for Carrying Out the Invention

In the following, a preferred embodiment of the present disclosure is described in detail with reference to the attached drawings. It is to be noted that the same reference sign is used to refer to components with substantially the same functional configuration herein and in the drawings to omit a redundant description.

Further, in this description and the drawings, components that have substantially the same functional configuration are sometimes distinguished from each other using different alphabets after the same reference sign. However, in a case where there is no need in particular to distinguish components that have substantially the same functional configuration, the same reference sign alone is attached to each of the components.

It is to be noted that the description is made in the following order.
[1. Background]
[2. Configuration]
   [2-1. Overall Configuration of Surgery Support System]
   [2-2. Configuration of Server]
[3. Operation]
[4. Modification Example]
   [4-1. Modification Example 1]
   [4-2. Modification Example 2]
   [4-3. Modification Example 3]
[5. Hardware Configuration Example]
[6. Conclusion]

### [1. Background]

Prior to describing one embodiment of the present disclosure, a background that has led to creation of one embodiment of the present disclosure will first be described. In an operating room, various cameras such as an endoscopic camera, a surgical field camera, and an operating room camera are used. Surgical images obtained by imaging performed by those cameras are displayed during surgery and may be recorded. It is to be noted that in this specification, in a case where there is no need to distinguish between a still image and a moving image, images may each be simply referred to as image. Also, in the present specification, the expression surgical image is used as an expression including a still image obtained during surgery or a moving image obtained during surgery.

Such a surgical image is not effectively utilized at present. Accordingly, the present disclosure proposes a mechanism of learning a plurality of surgical images (may also be referred to as surgical image group), and automatically predicting occurrence of a dangerous condition that may occur during surgery using a determiner obtained by the learning, which allows the surgical image to be effectively utilized. The dangerous condition that may occur during surgery may include, for example, an accidental symptom and an event that causes the accidental symptom. It is to be noted that in in this specification, various symptoms caused by hemorrhage, perforation, and a medical accident, a condition in which a large change in vital information occurs before and after medical practice (treatment), a condition in which a change in a surgical procedure is required, and other inconvenient symptoms caused by the medical practice are each collectively referred to as accidental symptom.

Incidentally, in a machine learning technique for performing such learning, it is desirable to perform learning using appropriate supervised data to obtain a more accurate determiner. As such supervised data, for example, it is conceivable to use label information that is obtained by labeling each still image or each frame in a moving image. Such label information is desirably prepared in accordance with performance of a desired determiner.

However, performing such labeling manually is costly, and is remarkably difficult particularly in a case where the surgical image included in the surgical image group is the moving image or the number of surgical images included in the surgical image group is large. In some cases, classification of the surgical image or addition of metadata to the surgical image is performed manually or by an automatic method, but no appropriate label information has been prepared that is able to be used as the supervised data for obtaining the above-described determiner by the learning.

Thus, the present embodiment has been created with the above-mentioned circumstances as a point of view. The surgery support system according to the present embodiment automatically generates the label information indicating the dangerous condition during surgery, the label information being able to be used as the supervised data. Further, the surgery support system according to the present embodiment may learn the surgical image group by using the generated label information as the supervised data, to thereby obtain the determiner. Thereafter, the surgery support system according to the present embodiment is able to predict occurrence of a dangerous condition in real time from the surgical image inputted during the surgery by using the thus obtained determiner. Hereinafter, in the present embodiment, a configuration and an operation that achieve the above effects will be described sequentially.

### [2. Configuration]

### [2-1. Overall Configuration of Surgery Support System]

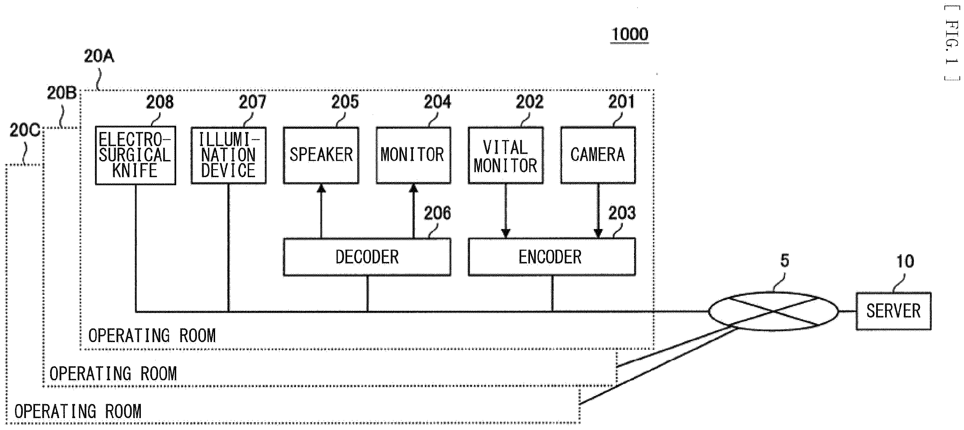

FIG. 1 is a block diagram illustrating a schematic configuration of a surgery support system 1000 according to an embodiment of the present disclosure. The surgery support system 1000 according to the present embodiment is present in a communication network 5, a server 10, and operating rooms 20A to 20C, and includes surgical devices which may be used during surgery. It is to be noted that, in this specification, a device which may be used during surgery is collectively referred to as surgical device, and not only a device of a medical application but also a device (e.g., a general-purpose device) which is not limited to a medical application is referred to as surgical device.

The communication network 5 is a wired or wireless transmission path for information transmitted from an apparatus coupled to the communication network 5. For example, the communication network 5 may include a public line network such as the Internet, a telephone line network, or a satellite communication network, various types of LAN (Local Area Network) and WAN (Wide Area Network) including Ethernet (registered trademark), and the like. Further, the communication network 5 may also include a leased line network such as IP-VPN (Internet Protocol-Virtual Private Network).

The server 10 is coupled to each of the surgical devices that are present in the respective operating rooms 20A to 20C via the communication network 5. The server 10 may be present in the operating rooms 20A to 20C or in a hospital where the operating rooms 20A to 20C are present, or may be present outside the hospital.

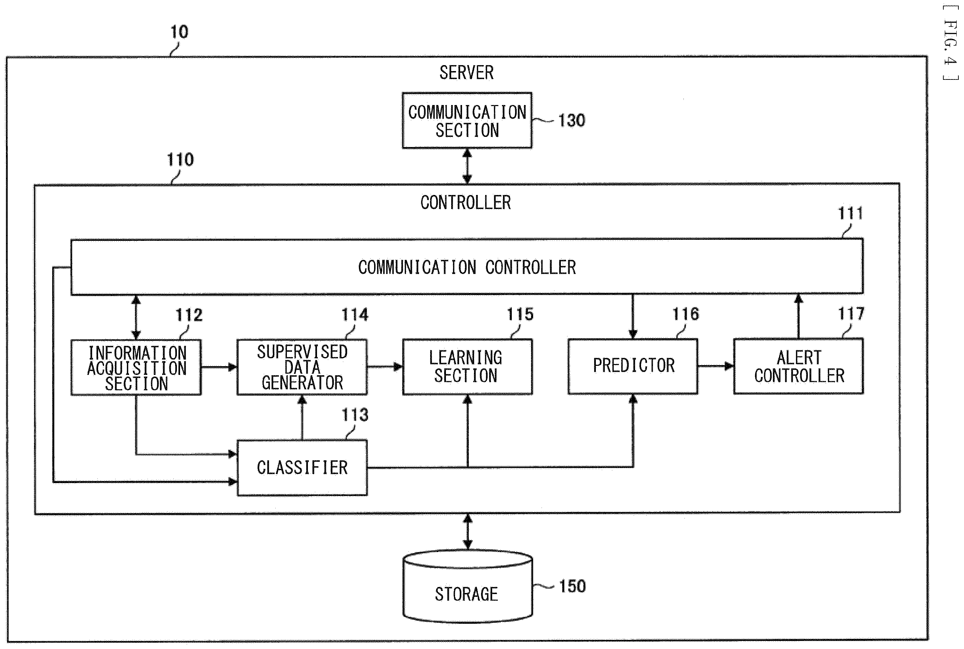

The server 10 receives surgical images (still images or moving images) from the surgical devices present in the operating rooms 20A to 20C and accumulates (records) the surgical images. Further, the server 10 also learns the accumulated surgical image group to thereby obtain a determiner. In addition, the server 10 predicts occurrence of a dangerous condition by using a surgical image received in real time as an input and using the previously obtained determiner. Further, in a case where the occurrence of the dangerous condition is predicted, the server 10 causes a surgical device to output an alert, the surgical device being present in an operating room from which the surgical image is acquired among the operating rooms 20A to 20C and functioning as an output section. It is to be noted that a detailed configuration of the server 10 will be described later with reference to FIG. 4.

Examples of the surgical devices present in each of the operating rooms 20A to 20C include, as illustrated in FIG. 1, for example, a camera 201, a vital monitor 202, an encoder 203, a monitor 204, a speaker 205, a decoder 206, an illumination device 207, and an electrosurgical knife (energy device) 208. Of those surgical devices, the monitor 204, the speaker 205, the illumination device 207, and the electrosurgical knife 208 may each function as an output section that outputs an alert warning that occurrence of a dangerous condition has been predicted by using image display, sound, light, or vibration. This allows visual, audible, or tactile alerts to be outputted to health care professionals such as operating surgeon and operating room staffs. It is to be noted that the surgical devices illustrated in FIG. 1 are exemplary, and other surgical devices may be included in the surgery support system 1000. For example, surgical devices such as a projector (an example of the output section), a bipolar, a surgical robot, etc. may be included in the surgery support system 1000. Further, although FIG. 1 only illustrates the surgical devices that are present in the operating room 20A, the surgical devices are also present similarly in the operating room 20B and the operating room 20C.

The camera 201 outputs a surgical image obtained by imaging to the encoder 203. The camera 201 may include, for example, an endoscopic camera, a surgical field camera, and an operating room camera. The endoscopic camera is inserted into, for example, a body cavity of a patient and acquires an image of a surgical site. Further, the surgical field camera acquires an image of the surgical site from an outside of the patient. Further, the operating room camera is provided, for example, on a ceiling of the operating room, and acquires an image of the entire operating room. It is to be noted that the camera 201 may include other cameras, and may include, for example, an electronic microscope or the like.

The vital monitor 202 outputs, to the encoder 203, an image (an example of a surgical image) obtained by visualizing vital information (e.g., a heart rate, a respiration (rate), a blood pressure, and a body temperature) of the patient measured during the surgery by an unillustrated vital information measurement device.

The encoder 203 (a live encoder) transmits the surgical image outputted from the camera 201 and the vital monitor 202 during the surgery to the server 10 in real time.

The monitor 204 functions as an output section, and displays (outputs) an image received from the server 10 by the decoder 206. The image to be displayed by the monitor 204 may include the surgical image acquired by the camera 201 present in the same operating room. Also, in a case where the server 10 predicts occurrence of a dangerous condition, the image to be displayed by the monitor 204 may include an alert, for example, the image may have an alert superimposed on the surgical image.

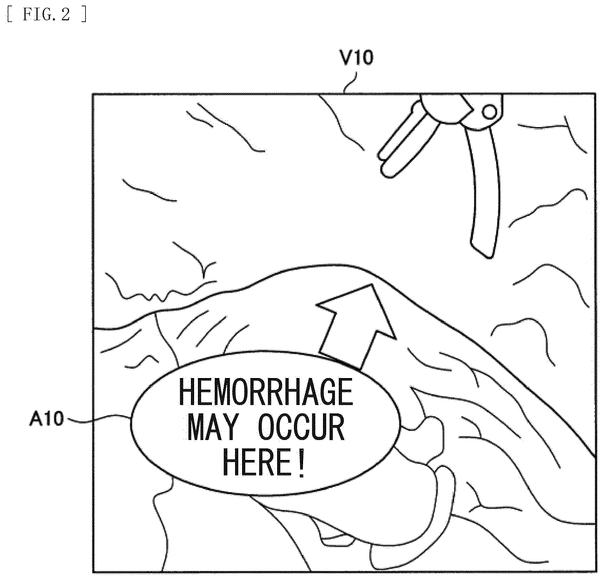

FIG. 2 illustrates an example of an image that includes an alert that is displayed on the monitor 204 in the case where the server 10 predicts the occurrence of the dangerous condition. An image VI0 illustrated in FIG. 2 is an image displayed on the monitor 204, and includes an alert A10 which warns that occurrence of hemorrhage (an example of the dangerous condition) has been predicted and a position at which the hemorrhage is predicted to occur. For example, a doctor confirms the alert A10 illustrated in FIG. 2 and performs surgery after recognizing a portion where the hemorrhage is likely to occur, which may make it possible to avoid the occurrence of hemorrhage.

Further, the monitor 204 may also display a manipulation screen for providing an instruction about the image display or for providing an instruction about an operation of a surgical device. In such cases, a touch panel may be provided on a display surface of the monitor 204 which makes it possible for a user to manipulate the touch panel.

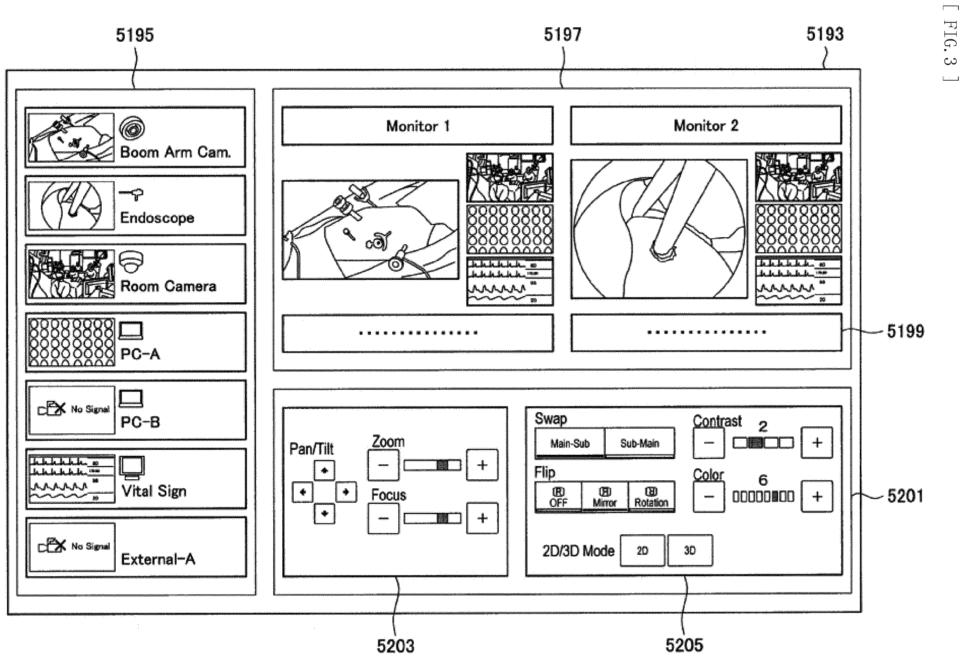

FIG. 3 is a diagram illustrating an example of a manipulation screen displayed on the monitor 204. FIG. 3 exemplarily illustrates a manipulation screen displayed on the monitor 204 in a case where at least two monitors 204 are provided in the operating room 20A as devices of output destinations. Referring to FIG. 3, a manipulation screen 5193 is provided with a transmission-source selection area 5195, a preview area 5197, and a control area 5201.

In the transmission-source selection area 5195, a transmission-source device provided in the surgery support system 1000 and a thumbnail screen indicating display information of the transmission-source device are displayed in association with each other. The user is able to select display information that the user wants to display on the monitor 204 from any of transmission-source devices displayed in the transmission-source selection area 5195.

The preview area 5197 displays screen previews of the two monitors 204 (a monitor 1 and a monitor 2) that are the devices of the output destinations. In the illustrated example, four images are PinP-displayed in one monitor 204. The four images correspond to the display information transmitted from the transmission-source device selected in the transmission-source selection area 5195. Of the four images, one is displayed relatively large as a main image and the remaining three are displayed relatively small as sub-images. The user is able to switch the main image and the sub images by selecting areas in which the four images are displayed as appropriate. In addition, a status display area 5199 is provided at a lower portion of the area where the four images are displayed, and a status related to surgery (e.g., elapsed time of surgery, physical information of a patient, etc.) may be displayed in the area as appropriate

The control area 5201 is provided with a transmission-source manipulation area 5203 in which GUI (Graphical User Interface) parts for performing manipulation on a transmission-source device are displayed, and an output-destination manipulation area 5205 in which GUI parts for performing manipulation on an output-destination device are displayed. In the illustrated example, the transmission-source manipulation area 5203 is provided with GUI parts for performing various types of manipulation (pan, tilt, and zoom) on a camera at a transmission-source device having an imaging function. The user is able to select these GUI parts as appropriate to manipulate operations of the camera at the transmission-source device. It is to be noted that, although illustration therefor is omitted, in a case where a transmission-source device selected in the transmission-source selection area 5195 is a recorder (i.e., in a case where images recorded in the recorder in the past are displayed in the preview area 5197), the transmission-source manipulation area 5203 may be provided with GUI parts for performing manipulation of playback, playback stop, rewind, forward, and the like of the images.

In addition, the output-destination manipulation area 5205 is provided with GUI parts for performing various types of manipulation (swap, flip, color adjustment, contrast adjustment, and switching between 2D display and 3D display) on displays at the monitor 204 which is an output-destination device. The user is able to select these GUI parts as appropriate to manipulate displays on the monitor 204.

It is to be noted that the manipulation screen displayed in the monitor 204 is not limited to the illustrated example, and the user may be able to perform manipulation input for each device provided in the surgery support system 1000 via the monitor 204.

The speaker 205 functions as an output section and outputs an audio received from the server 10 by the decoder 206. For example, in the case where the server 10 predicts the occurrence of the dangerous condition, the speaker 205 outputs the audio (an example of an alert) warning that the occurrence of the dangerous condition has been predicted.

The decoder 206 receives the image and the audio from the server 10 and outputs the image and the audio to the monitor 204 and the speaker 205, respectively.

The illumination device 207 is an illumination device to be used in an operating room, such as a shadowless lamp. The illumination device 207 according to the present embodiment is coupled to the server 10 via the communication network 5 as illustrated in FIG. 1. In addition, the illumination device 207 according to the present embodiment functions as an output section, and outputs an alert warning that the occurrence of the dangerous condition has been predicted, in accordance with a control signal received from the server 10. For example, the illumination device 207 may output the alert by outputting light having a predetermined color or by making lighting patterns different from normal.

The electrosurgical knife 208 is a surgical tool used for surgery, and is able to stop hemorrhage at the same time as surgical interruption, for example, by applying a high-frequency current to a human body. Further, the electrosurgical knife 208 according to the present embodiment is coupled to the server 10 via the communication network 5 as illustrated in FIG. 1. The electrosurgical knife 208 according to the present embodiment functions as an output section, and outputs an alert warning that the occurrence of the dangerous condition has been predicted, in accordance with a control signal received from the server 10. For example, the electrosurgical knife 208 may output an alert by vibrating a handle section.

As described above, in the case where the server 10 predicts that the occurrence of the dangerous condition, an alert warning that the occurrence of the dangerous condition has been predicted is outputted. As a result, the dangerous condition may be avoided, for example, by the operating surgeon ceasing to take actions leading to a medical accident or performing surgery after recognizing a portion at which hemorrhage may occur.

### [2-2. Configuration of Server]

Heretofore, the configuration of the surgery support system 1000 according to the present embodiment has been described. Subsequently, a more detailed configuration of the server 10 illustrated in FIG. 1 will be described referring to FIG. 4. FIG. 4 is a block diagram illustrating a configuration example of the server 10 according to the present embodiment. As illustrated in FIG. 4, the server 10 is an information processing apparatus including a controller 110, a communication section 130, and a storage 150.

The controller 110 functions as an arithmetic processing unit and a control unit, and controls an overall operation of the server 10 in accordance with various programs. Further, the controller 110 functions as, as illustrated in FIG. 4, a communication controller 111, an information acquisition section 112, a classifier 113, a supervised data generator 114, a learning section 115, a predictor 116, and an alert controller 117.

The communication controller 111 controls communication performed by the communication section 130 with another apparatus. For example, the communication controller 111 controls the communication section 130 to receive a surgical image from the encoder 203 illustrated in FIG. 1. Further, the communication controller 111 controls the communication section 130 to receive surgery attribute information in accordance with an instruction issued by the information acquisition section 112 to be described later. Still further, the communication controller 111 controls the communication section 130 to transmit an image, an audio, a control signal, and the like for outputting the above-described alert to the surgical devices present in the respective operating rooms 20A to 20C illustrated in FIG. 1 in accordance with an instruction issued by the alert controller 117 to be described later.

The information acquisition section 112 acquires (collects) surgery attribute information (meta information) corresponding to a surgical image received from the encoder 203. The information acquisition section 112 may output an instruction for acquiring the surgery attribute information to the communication controller 111 and acquire, from the communication controller 111, the surgery attribute information which has been received by the communication controller 111 controlling the communication section 130 in accordance with the instruction.

For example, the information acquisition section 112 acquires pieces of surgery attribute information not only from the surgical devices included in the operating rooms 20A to 20C illustrated in FIG. 1 but also from unillustrated databases, systems, and the like inside and outside the hospital, and associates the pieces of surgery attribute information with respective surgical images. The following is a description of an example of the surgery attribute information that the information acquisition section 112 acquires.

The surgery attribute information may include patient information such as the age, sex, race, condition, etc. of a patient, for example. The patient information may be acquired from, for example, HIS (Hospital Information System), EMR (Electronic Medical Record, also referred to as electronic medical record), or the like.

The surgery attribute information may also include doctor information such as identification information of a doctor, a name of the doctor, a medical office to which the doctor belongs, a graduating school of the doctor, etc. The doctor information may be acquired, for example, from RIS (Radiology Information System, also referred to as order system), a surgical planning system, an anesthesia machine system, or an internet-based doctor information site.

The surgery attribute information may also include operative method information related to an operative method such as a name of the operative method (e.g., esophagectomy, total gastrectomy, small intestine malignant tumor surgery, partial hepatectomy, distal pancreatectomy, lobectomy, TAPVR surgery, craniotomy for removal of hematoma, etc.), a technique procedure, a time allocation of the technique procedure, etc. The operative method information may be acquired from, for example, RIS, an operative method database in the hospital, or an operative method information site on the Internet.

The surgery attribute information may also include surgical device information indicating a condition (e.g., usage state, status, etc.) of a surgical device such as the electrosurgical knife 208, a surgical robot, etc. For example, in a case of the surgical robot, the surgical device information may include a condition of a joint of an arm included in the robot, an attitude of the arm, and the like. In a case of the electrosurgical knife, the surgical device information may include a state of ON/OFF manipulation. The surgical device information may be acquired from each of the surgical devices present in the operating rooms 20A to 20C.

The surgery attribute information acquired by the information acquisition section 112 is associated with a surgical image and outputted to the classifier 113 and the supervised data generator 114.

The classifier 113 classifies the surgical image on the basis of the surgery attribute information. The classifier 113 may classify the surgical image for each operative method on the basis of the operative method information included in the surgery attribute information, for example. However, the method of classifying the surgical image by the classifier 113 is not limited to such an example, and it is possible to perform a more diverse classification on the basis of various kinds of information included in the surgery attribute information.

The classifier 113 outputs the classified surgical image and information related to the classification of the surgical image to the supervised data generator 114 to be described later. Such a configuration allows the supervised data generator 114 to more efficiently generate label information that is to be supervised data for each surgical image classified by the classifier 113.

The classifier 113 also outputs a plurality of surgical image groups obtained by classifying a plurality of surgical images (still images or moving images) and information related to the classification of the plurality of surgical image groups to the learning section 115 to be described later. Such a configuration allows the learning section 115 to perform learning for each surgical image group classified by the classifier 113, thereby improving efficiency of the learning and improving performance of a determiner to be obtained. It is to be noted that the plurality of surgical image groups classified by the classifier 113 may be stored in the storage 150.

Further, the classifier 113 outputs the classified surgical image and the information related to the classification of the surgical image to the predictor 116 to be described later. Such a configuration allows the predictor 116 to select a determiner based on the classification of the surgical image and perform a prediction, thereby improving prediction accuracy.

The supervised data generator 114 generates label information indicating a dangerous condition during surgery on the basis of the surgical image classified by the classifier 113 and the surgery attribute information acquired by the information acquisition section 112. The label information generated by the supervised data generator 114 is used as supervised data by the learning section 115 to be described later.

For example, the supervised data generator 114 may generate the label information by performing a hemorrhage detection to detect hemorrhage, a reworking detection to detect reworking due to a medical accident, a hemostasis detection to detect execution of hemostasis, and the like. For example, in a case where the hemorrhage is detected, label information indicating the hemorrhage (an example of the dangerous condition) may be generated and added to a frame in which the hemorrhage has been detected. Further, in a case where the reworking is detected, label information indicating a medical accident (an example of the dangerous condition) may be generated and added to a frame in which the reworking is detected or a frame corresponding to a medical accident that is to be a cause of the reworking. Also, in a case where the execution of the hemostasis is detected, label information indicating the hemorrhage (an example of the dangerous condition) may be generated and added to a frame in which the execution of the hemostasis has been detected (or, if detectable, to a frame of during hemorrhage).

For example, the supervised data generator 114 may perform the hemorrhage detection by detecting a feature amount of a red color, a liquid, and the like from the surgical image by image recognition.

Further, the supervised data generator 114 may perform the reworking detection by detecting a scene change, for example. The scene change may be performed by a method of detecting a change in a pattern from the surgical image, a method of detecting insertion and removal of an endoscope from the surgical image, or a method of detecting a change in a surgical instrument recognized in the surgical image.

Further, the surgical device information acquired by the information acquisition section 112 may be used to detect the scene change. For example, as the surgical device information, a use state and a status change of the electrosurgical knife 208 or the bipolar, a status of the surgical robot, a change of forceps during use, and the like may be used.

The operative method information acquired by the information acquisition section 112 may be used to detect the scene change. For example, as the operative method information, information related to the time allocation of the technique procedure for each operative method may be used. It should be noted that these pieces of information may differ depending on whether the patient is an adult or a child, or a degree of obesity or the like, and therefore, the time allocation of the surgical procedure may be selectively used or corrected by using the patient information acquired by the information acquisition section 112.

For example, the supervised data generator 114 may determine that the reworking has occurred in a case where the difference between the time allocation of the technique procedure included in the operative method information and the time allocation of the technique procedure estimated from the surgical device information is large. Further, the supervised data generator 114 may detect, as the frame corresponding to a medical accident that is to be a cause of the reworking, a frame in which a difference has started to occur between the time allocation of the technique procedure included in the operative method information and the time allocation of the technique procedure estimated from the surgical device information.

Further, the supervised data generator 114 may detect that the hemostasis is being performed by detecting a feature of a pre-learned surgical instrument for the hemostasis (e.g., a needle and a thread for ligation, the electrosurgical knife 208, etc.). For example, in a case where the electrosurgical knife 208 is in a coagulation mode, the supervised data generator 114 is able to detect that the hemostasis is being performed.

It is to be noted that the method in which the supervised data generator 114 generates the label information indicating the dangerous condition during surgery is not limited to the examples described above. For example, the supervised data generator 114 may determine that a medical accident has occurred and generate label information indicating the medical accident if it is detected that the number of doctors included (gathered) in the surgical image is larger than normal.

The learning section 115 generates (acquires) a determiner (a learned model) by learning the surgical image group classified by the classifier 113 using, as the supervised data, the label information generated by the supervised data generator 114. A method of learning by the learning section 115 is not particularly limited, and, for example, the learning may be performed by preparing learning data in which label information and a surgical image group are associated with each other, and inputting the learning data to a computational model based on a multi-layer neural network. Alternatively, a method based on DNN (Deep Neural Network) such as CNN (Convolutional Neural Network), 3D-CNN, RNN (Recurrent Neural Network) or the like may also be used.

The determiner generated by the learning section 115 is used by the predictor 116 to be described later to perform a prediction of occurrence of a dangerous condition. Thus, the learning section 115 learns, as a surgical image leading to the occurrence of the dangerous condition, a surgical image of a frame prior to a frame to which the label information indicating the dangerous condition is added among the surgical image group. Such a configuration allows the determiner generated by the learning section 115 to be used for the prediction of the occurrence of the dangerous condition prior to the occurrence of the dangerous condition.

The learning section 115 may generate a plurality of determiners. As described above, the classifier 113 may classify the plurality of surgical images into the plurality of surgical image groups; therefore, the learning section 115 may generate a determiner for each classified surgical image group. That is, the same number of determiners as the number of surgical image groups classified by the classifier 113 may be generated.

The plurality of determiners generated by the learning section 115 is stored in the storage 150 in association with the information related to the classification of the surgical image groups used for generating the respective determiners.

The predictor 116 performs a prediction of the occurrence of the dangerous condition by using a surgical image (a still image or a moving image) classified by the classifier 113 as an input and using the determiner stored in the storage 150.

As described above, the plurality of determiners is stored in the storage 150. Accordingly, the predictor 116 may select a determiner to be used for the prediction from the plurality of determiners stored in the storage 150 on the basis of the classification of the surgical image performed by the classifier 113.

Such a configuration may cause a determiner that is more suitable for current surgery to be selected and may improve the prediction accuracy of the dangerous condition. It is to be noted that such selection of the determiner may be performed on a frame-by-frame basis, or may be performed only at the beginning of surgery and the same determiner may be used during the surgery.

Further, in a case where the occurrence of the dangerous condition is predicted, the predictor 116 generates information related to the prediction (hereinafter, referred to as prediction information), such as a type of the dangerous condition (hemorrhage, perforation, a medical accident, or the like), a degree of risk of the dangerous condition, a position at which the dangerous condition is predicted to occur, or the like. Further, in the case where the occurrence of the dangerous condition is predicted, the predictor 116 provides the generated prediction information to the alert controller 117.

The alert controller 117 causes, in the case where the occurrence of the dangerous condition is predicted by the predictor 116, an alert to be outputted on the basis of the prediction information provided by the predictor 116. As described above, alerts are outputted by the surgical devices (the monitor 204, the speaker 205, the illumination device 207, and the electrosurgical knife 20 in the example illustrated in FIG. 1) that are present in an operating room where the dangerous condition is predicted to occur and function as the output sections. The alert controller 117 may generate an image, an audio, and a control signal for those output sections to output the alerts, and provide the communication controller 111 with the generated image, audio, and image signal, thereby causing the alerts to be outputted.

The alert controller 117 may cause different alerts to be outputted depending on the prediction information. Further, the alert controller 117 may also cause an output section (a surgical device) corresponding to the prediction information to output an alert.

For example, in a case where the prediction information includes information of the type of the dangerous condition, the alert controller 117 may cause an alert including information of the type of the dangerous condition to be outputted. For example, the alert controller 117 may generate an image in which an alert indicating the information of the type of the dangerous condition is combined with a surgical image, and may cause the monitor 204 to display the combined image. Further, the alert controller 117 may cause the speaker 205 to output an audio including the information of the type of the dangerous condition. In addition, the alert controller 117 may cause the illumination device 207 to output light beams having different colors depending on the type of the dangerous condition. Still further, the alert controller 117 may cause the electrosurgical knife 208 to have different vibration patterns depending on the type of the dangerous condition.

Such a configuration enables the operating surgeon to grasp the type of the dangerous condition that is predicted to occur, thereby making it easier to avoid the dangerous condition.

Further, in a case where the prediction information includes information of the degree of risk of the dangerous condition, the alert controller 117 may cause an alert corresponding to the degree of risk of the dangerous condition to be outputted. For example, in a case where the degree of risk is high, the alert controller 117 may generate an image in which a more prominent alert than a case where the degree of risk is low is combined with a surgical image, and may cause the monitor 204 to display the combined image. Further, the alert controller 117 may also change a display size or a color of the alert. Still further, in the case where the degree of risk is high, the alert controller 117 may increase a volume of the alert outputted from the speaker 205 as compared to the case where the degree of risk is low. In addition, in the case where the degree of risk is high, the alert controller 117 may increase an intensity of light outputted to the illumination device 207 as compared to the case where the degree of risk is low. Moreover, in the case where the degree of risk is high, the alert controller 117 may increase a vibration intensity of the electrosurgical knife 208 as compared to the case where the degree of risk is low.

Such a configuration enables the operating surgeon to be more vigorously alerted, for example, in the case where the risk of the dangerous condition predicted to occur is higher.

Further, in a case where the prediction information includes information of a position at which the dangerous condition is predicted to occur, the alert controller 117 may generate an image including an alert indicating the position at which the dangerous condition is predicted to occur, and may cause the monitor 204 to display the image. In addition, the alert controller 117 may also control a projector (not illustrated) to cause the alert to be projected at the position at which the dangerous condition is predicted to occur.

Such a configuration enables the operating surgeon to grasp the position at which the dangerous condition is predicted to occur, thereby making it easier to avoid the dangerous condition.

As described above, although examples of the alert to be outputted by the alert controller 117 have been described, the present technology is not limited to such examples, and an alert other than the above may be outputted.

The communication section 130 is a communication module for transmitting and receiving data to and from another apparatus via wire or radio in accordance with control of the communication controller 111. The communication section 130 communicates wirelessly with an external device directly or via a network access point by a scheme of wired LAN (Local Area Network), wireless LAN, Wi-Fi (Wireless Fidelity, registered trademark), infrared communication, Bluetooth (registered trademark), near-field/contactless communication, or the like.

The storage 150 stores a program and a parameter for each component of the server 10 to function. For example, the storage 150 stores the plurality of surgical image groups classified by the classifier 113 and the information related to the classification of the plurality of surgical image groups. Further, the storage 150 stores the plurality of determiners generated by the learning section 115. As described above, the determiner is generated for each classified surgical image group; therefore, the storage 150 stores the determiner and the information related to the classification of the surgical image group of the determiner in association with each other.

### [3. Operation]

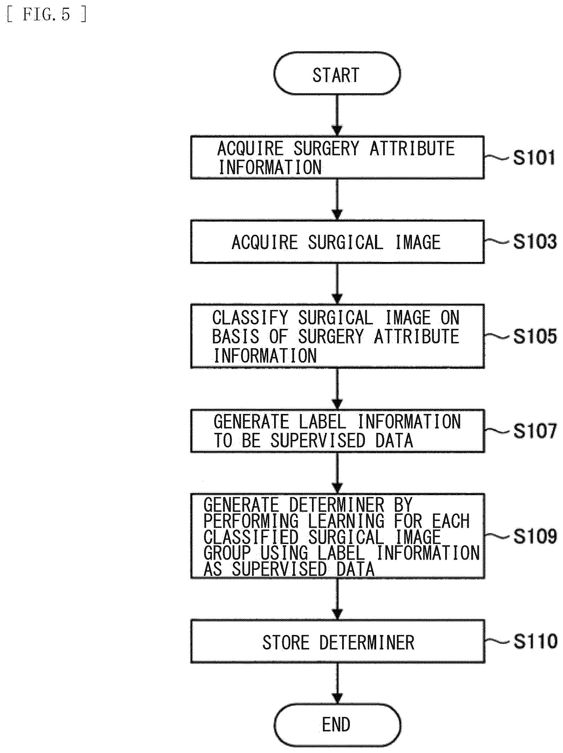

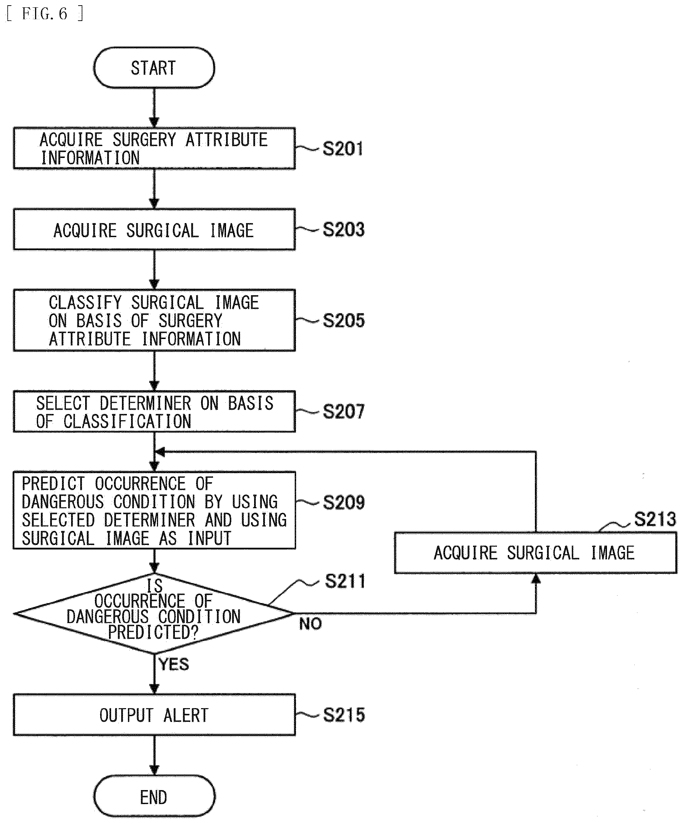

The respective configurations of the surgery support system 1000 and the server 10 according to the present embodiment have been described above. Subsequently, an operation example of the surgery support system 1000 according to the present embodiment will be described. It is to be noted that in the following, an operation related to learning will be described first with reference to FIG. 5, and then an operation related to a prediction of a dangerous condition performed during surgery will be described with reference to FIG. 6.

FIG. 5 is a flowchart illustrating an example of an operation of the surgery support system 1000 related to learning. It is be noted that a process illustrated in FIG. 5 may be performed in advance, for example, prior to a process related to a prediction of a dangerous condition, which will be described later referring to FIG. 6.

First, the information acquisition section 112 acquires surgery attribute information (S101). Further, the communication section 130 receives (acquires) a surgical image from the encoder 203 (SI03). It is to be noted that step S101 and step S103 may be performed in parallel.

Subsequently, the classifier 113 classifies, on the basis of the surgery attribute information acquired in step S101, the surgical image acquired in step S103 (S105). Subsequently, the supervised data generator 114 generates, on the basis of the surgery attribute information acquired in step S101 and the surgical image classified in step S105, label information to be supervised data (S107).

Subsequently, the learning section 115 generates a determiner by performing learning for each surgical image group classified in step S105 using, as the supervised data, the label information generated in step S107 (SI09), and causes the storage 150 to store the determiner (S110).

The operation related to learning has been described above. Subsequently, the operation related to a prediction of a dangerous condition performed during surgery will be described. FIG. 6 is a flowchart illustrating an example of an operation of the surgery support system 1000 related to a prediction of a dangerous condition. It is be noted that a process illustrated in FIG. 6 is performed, for example, after the process described with reference to FIG. 5 is performed and the determiner is stored in the storage 150.

First, the information acquisition section 112 acquires surgery attribute information (S201). Further, the communication section 130 receives (acquires) a surgical image from the encoder 203 (S203). It is to be noted that step S201 and step S203 may be performed in parallel.

Subsequently, the classifier 113 classifies, on the basis of the surgery attribute information acquired in step S201, the surgical image acquired in step S203 (S205). Subsequently, the predictor 116 selects, on the basis of the classification performed in step S207, a determiner to be used for a prediction from among the plurality of determiners stored in the storage 150 (S207).

Further, the predictor 116 performs a prediction of occurrence of a dangerous condition by using the determiner selected in step S207 and using the surgical image acquired in step S203 as an input (S209).

As a result of the prediction of step S209, in a case where the occurrence of the dangerous condition is not predicted (NO in S211), the communication section 130 receives (acquires) a surgical image from the encoder 203 again (S213). Then, the process returns to step S209, and the prediction of the occurrence of the dangerous condition is performed by using the surgical image acquired in step S213 as an input.

In contrast, as a result of the prediction of step S209, in a case where the occurrence of the dangerous condition is predicted (YES in S211), the output section such as the monitor 204, the speaker 205, the illumination device 207, or the electrosurgical knife 208 outputs an alert in accordance with control of the alert controller 117 (S211).

### [4. Modification Example]

The configuration examples and the operation examples according to the present embodiment have been described above. Hereinafter, modification examples of the present embodiment will be described. It is to be noted that the modification examples described below may be applied to the present embodiment independently or may be applied to the present embodiment in combination. Further, the present modification examples may be applied instead of the configurations described in the present embodiment, or may be additionally applied to the configurations described in the present embodiment.

### [4-1. Modification Example 1]

The respective configurations of FIG. 1 and FIG. 4 described in the above embodiment are examples, and the present technology is not limited to such examples. For example, some or all the functions of the server 10 described in the above embodiment may be provided in another apparatus. For example, the function related to the learning such as the supervised data generator 114, the learning section 115, or the like and the function related to the prediction of the occurrence of the dangerous condition such as the predictor 116, the alert controller 117, or the like may be provided in different apparatuses. Thereafter, the determiner obtained by the learning may be provided from the apparatus that performs the learning to the apparatus that performs the prediction.

Further, the function related to the prediction described above may be provided in a surgical device such as the camera 201, the vital monitor 202, the encoder 203, the monitor 204, the speaker 205, the decoder 206, the illumination device 207, or the electrosurgical knife 208 present in the operating rooms 20A to 20C illustrated in FIG. 1.

Further, the generation of the image to be displayed on the monitor 204 as an alert may not necessarily be performed by the server 10. For example, the monitor 204 may directly receive and display a surgical image acquired by the camera 201 in the same surgery, and, in a case where a control signal or the like related to the alert is received from the server 10, may generate and display an image in which the alert and the surgical image are combined.

### [4-2. Modification Example 2]

Further, in the above embodiment, the example has been described in which the surgical image group including the surgical images provided from the surgical device present in the operating room is used for the learning; however, the present technology is not limited to such an example. For example, a surgical image group recorded in an external database or the like or corresponding surgery attribute information may be provided to the server 10 and used for the learning.

### [4-3. Modification Example 3]

Further, in the above embodiment, the example has been described in which the determiner is generated by learning in advance and then the prediction is performed; however, the present technology is not limited to such an example. For example, the surgery attribute information or the surgical image acquired in the process of the prediction described with reference to FIG. 6 may be used for the learning, and the determiner may be updated at any time.

### [5. Hardware Configuration Example]

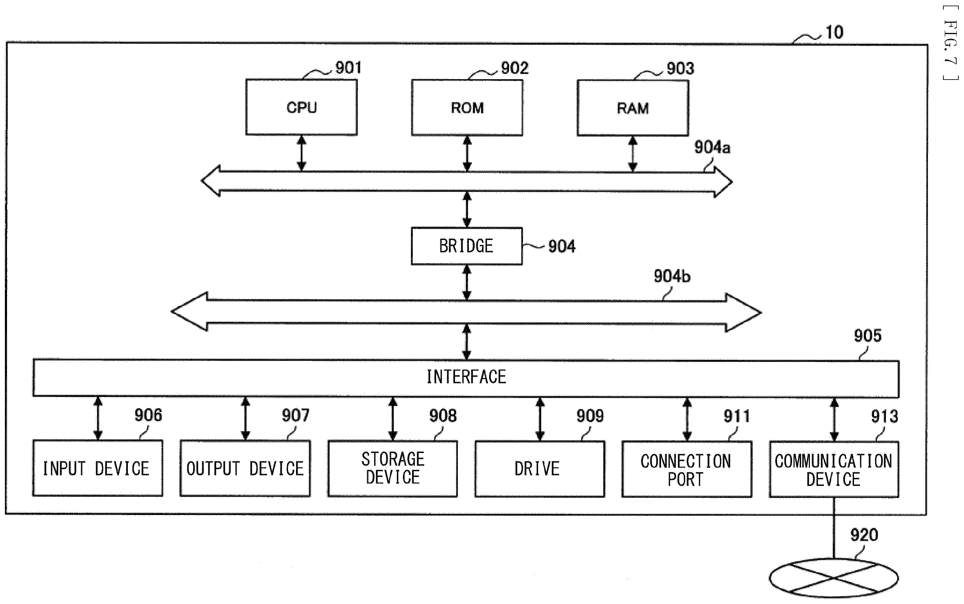

The embodiment of the present disclosure has been described above. Finally, referring to FIG. 7, a hardware configuration of the information processing apparatus according to an embodiment of the present disclosure will be described. FIG. 7 is a block diagram illustrating an example of a hardware configuration of the server 10 according to an embodiment of the present disclosure. The information processing by the server 10 according to an embodiment of the present disclosure is achieved in cooperation with hardware described below and software.

As illustrated in FIG. 7, the server 10 includes a CPU (Central Processing Unit) 901, a ROM (Read Only Memory) 902, a RAM (Random Access Memory) 903, and a host bus 904a. Further, the server 10 includes a bridge 904, an external bus 904b, an interface 905, an input device 906, an output device 907, a storage device 908, a drive 909, a connection port 911, and a communication device 913. The server 10 may have a processing circuit, such as DSP or ASIC, instead of or in addition to the CPU 901.

The CPU 901 functions as an arithmetic processing unit and a control unit, and controls an overall operation of the server 10 in accordance with various programs. Further, the CPU 901 may be a microprocessor. The ROM 902 stores a program and an arithmetic parameter to be used by the CPU 901. The RAM 903 temporarily stores a program used in executing the CPU 901, a parameter that appropriately changes in executing the same, and the like. The CPU 901 may serve as the controller 110, for example.

The CPU 901, the ROM 902, and the RAM 903 are interconnected via the host bus 904a including a CPU bus and the like. The host bus 904a is connected via the bridge 904 to the external bus 904b such as a PCI (Peripheral Component Interconnect/Interface) bus. It is to be noted that the host bus 904a, the bridge 904, and the external bus 904b do not necessarily have to be configured to be separated from each other, and the functions thereof may be implemented in one bus.

The input device 906 is achieved by a device to which information is inputted by a user, such as a mouse, a keyboard, a touch panel, a button, a microphone, a switch, a lever, and the like. Further, the input device 906 may be, for example, a remote-control device using infrared rays or other radio waves, or may be an externally connected device such as a mobile phone or PDA compatible with manipulation of the server 10. In addition, the input device 906 may include, for example, an input control circuit that generates an input signal on the basis of information inputted by the user using the above-mentioned input means, and outputs the input signal to the CPU 901. The user of the server 10 is able to input various types of data to the server 10 or provide the server 10 with an instruction on a processing operation by manipulating the input device 906.

The output device 907 includes a device that is able to visually or audibly notifying the user of acquired information. Examples of such a device include a display device such as a CRT display device, a liquid crystal display device, a plasma display device, an EL display device, or a lamp, an audio output device such as a speaker or headphones, a printer device, and the like. The output device 907 outputs, for example, a result obtained by various processes performed by the server 10. Specifically, the display device visually displays the result obtained by the various processes performed by the server 10 in a variety of formats, such as text, images, tables, graphs, and the like. Meanwhile, the audio output device converts an audio signal including reproduced audio data, acoustic data, and the like into an analog signal, and aurally outputs the analog signal.

The storage device 908 is a device for data storage serving as an example of the storage of the server 10. The storage device 908 is achieved by, for example, a magnetic storage device such as an HDD, a semiconductor storage device, an optical storage device, a magneto-optical storage device, or the like. The storage device 908 may include a storage medium, a recording device that records data on the storage medium, a read device that reads data from the storage medium, and a deletion device that deletes data recorded on the storage medium. The storage device 908 stores a program or various types of data to be executed by the CPU 901, and various types of data acquired from the outside. The storage device 908 may serve as the storage 150, for example.

The drive 909 is a reader/writer for a storage medium, and is built in or externally attached to the server 10. The drive 909 reads information recorded on a removable storage medium such as a magnetic disk, an optical disc, a magneto-optical disk, or a semiconductor memory, and outputs the read information to the RAM 903. In addition, the drive 909 is also able to write information into the removable storage medium.

The connection port 911 is an interface connected to an external device and is a connection port to an external device that is able to transmit data, for example, by USB (Universal Serial Bus).

The communication device 913 is, for example, a communication interface including a communication device to be connected to the network 920 or the like. The communication device 913 may be, for example, a communication card or the like for wired or wireless LAN (Local Area Network), LTE (Long Term Evolution), Bluetooth (registered trademark), or WUSB (Wireless USB). Further, the communication device 913 may be a router for optical communication, a router for ADSL (Asymmetric Digital Subscriber Line), or a modem for any type of communication. The communication device 913 is able to transmit and receive signals or the like to and from the Internet or another communication device in accordance with a predetermined protocol such as TCP/IP, for example. The communication device 913 may serve as the communication section 130, for example.

It is to be noted that the network 920 is a wired or wireless transmission path for information transmitted from an apparatus coupled to the network 920. For example, the network 920 may include a public line network such as the Internet, a telephone line network, or a satellite communication network, various types of LAN (Local Area Network) and WAN (Wide Area Network) including Ethernet (registered trademark), and the like. Further, the network 920 may also include a leased line network such as IP-VPN (Internet Protocol-Virtual Private Network).

As described above, an example of the hardware configuration that is able to achieve the functions of the server 10 according to an embodiment of the present disclosure has been described. Each of the above components may be implemented using a general-purpose member, or may be implemented using hardware that is specialized to the function of each component. Accordingly, it is possible to change hardware configuration to be utilized as appropriate in accordance with the technical levels at which the embodiments of the present disclosure are carried out.

It is to be noted that it is possible to manufacture a computer program for achieving the functions of the server 10 according to an embodiment of the present disclosure as described above and to mount the computer program on PC or the like. In addition, it is possible to provide a computer-readable recording medium having such a program stored therein. The recording medium is, for example, a magnetic disk, an optical disc, a magneto-optical disk, a flash memory, or the like. In addition, the program described above may be distributed, for example, through a network without using a recording medium.

### [6. Conclusion]

As described above, according to an embodiment of the present disclosure, it becomes possible to predict occurrence of a dangerous condition by utilizing a surgical image. In addition, in the case the occurrence of the dangerous condition is predicted, it is possible to output an alert prior to the occurrence of the dangerous condition, and the dangerous condition may be avoided by the operating surgeon ceasing to take actions leading to a medical accident or performing surgery after recognizing a portion at which hemorrhage may occur

As a result, a degree of invasion on a patient is reduced and a surgery time is shortened. In addition, a quality of life of the patient is increased and a degree of satisfaction of the patient is increased, leading to an increase in the number of customers of a hospital. It is also expected that improved utilization of operating rooms will improve profitability of the hospital. In addition, it is expected that the reduction of a risk of accidental symptoms will alleviate tensions of doctors, raise a degree of job satisfaction of the doctors, prevent turnover, and reduce labor costs.

Although the detailed description has been made above on the preferred embodiment of the present disclosure with reference to the attached drawings, the technical scope of the present disclosure is not limited to such an example. It is obvious that a variety of alterations and modifications within the scope of the technical idea according to the claims would occur to those having ordinary knowledge in the art to which the present disclosure pertains and it is, of course, understood that these also belong to the technical scope of the present disclosure.

For example, the steps of the above embodiment are not necessarily performed in time series along the order indicated in the flowchart. For example, the steps of the process of the above embodiment may be performed in an order different from the order indicated in the flowchart or performed in parallel.

In addition, the effects described herein are merely explanatory and illustrative and not limitative. That is, the technology according to the present disclosure may exhibit other effects obvious to those skilled in the art from the description herein in addition to the above-described effects or instead of the above-described effects.

It is to be noted that the following configurations also belong to the technical scope of the present disclosure.
(1) A surgery support system including:
   a storage that stores a determiner, the determiner being obtained by learning a surgical image group using, as supervised data, label information indicating a dangerous condition during surgery; and
   a predictor that performs a prediction of occurrence of a dangerous condition by using a surgical image as an input and using the determiner.
(2) The surgery support system according to (1), in which
   the storage stores a plurality of the determiners, and
   the predictor performs the prediction by using, out of the plurality of determiners, the determiner corresponding to the surgical image.
(3) The surgery support system according to (2), in which the determiner to be used for the prediction is selected on a basis of a classification of the surgical image.
(4) The surgery support system according to (3), in which the surgical image is classified on a basis of operative method information related to an operative method.
(5) The surgery support system according to any one of (2) to (4), further including
   a learning section that generates the determiner by performing learning for each surgical image group that has been classified.
(6) The surgery support system according to (5), further including
   a supervised data generator that generates the label information.
(7) The surgery support system according to (6), in which the supervised data generator generates the label information by performing at least one of a hemorrhage detection to detect hemorrhage, a reworking detection to detect reworking, or a hemostasis detection to detect execution of hemostasis.
(8) The surgery support system according to any one of (1) to (7), further including
   an output section that outputs an alert in a case where the occurrence of the dangerous condition is predicted by the predictor, the alert warning that the occurrence of the dangerous condition has been predicted.
(9) The surgery support system according to (8), in which
   in the case where the occurrence of the dangerous condition is predicted, the predictor generates prediction information related to a prediction, and
   the output section outputs the alert depending on the prediction information.
(10) The surgery support system according to (9), in which
   the surgery support system includes a plurality of the output sections, and
   the alert is outputted from, out of the plurality of output sections, the output section corresponding to the prediction information.
(11) The surgery support system according to (9) or (10), in which the prediction information includes at least one piece of information selected from a type of the dangerous condition the occurrence of which has been predicted, a degree of risk of the dangerous condition, and a position at which the dangerous condition is predicted to occur.
(12) The surgery support system according to any one of (1) to (11), in which the dangerous condition includes an accidental symptom or an event that causes the accidental symptom.
(13) The surgery support system according to any one of (1) to (12), in which the surgical image group includes a plurality of moving images, and the surgical image is a moving image.
(14) An information processing apparatus including:
   a storage that stores a determiner, the determiner being obtained by learning a surgical image group using, as supervised data, label information indicating a dangerous condition during surgery; and
   a predictor that performs a prediction of occurrence of a dangerous condition by using a surgical image as an input and using the determiner.
(15) A program causing a computer to implement
   a function of storing a determiner, the determiner being obtained by learning a surgical image group using, as supervised data, label information indicating a dangerous condition during surgery, and
   a function of performing a prediction of occurrence of a dangerous condition by using a surgical image as an input and using the determiner.

### Reference Signs List

- 10: server
- 20A to 20C: operating room
- 110: controller
- 111: communication controller
- 112: information acquisition section
- 113: classifier
- 114: supervised data generator
- 115: learning section
- 116: predictor
- 117: alert controller
- 130: communication section
- 150: storage
- 201: camera
- 202: vital monitor
- 203: encoder
- 204: monitor
- 205: speaker
- 206: decoder
- 207: illumination device
- 208: electrosurgical knife
- 1000: surgery support system

## Claims

1. A surgery support system comprising:
a storage that stores a determiner, the determiner being obtained by learning a surgical image group using, as supervised data, label information indicating a dangerous condition during surgery; and
a predictor that performs a prediction of occurrence of a dangerous condition by using a surgical image as an input and using the determiner.

2. The surgery support system according to claim 1, wherein
the storage stores a plurality of the determiners, and
the predictor performs the prediction by using, out of the plurality of determiners, the determiner corresponding to the surgical image.

3. The surgery support system according to claim 2, wherein the determiner to be used for the prediction is selected on a basis of a classification of the surgical image.

4. The surgery support system according to claim 3, wherein the surgical image is classified on a basis of operative method information related to an operative method.

5. The surgery support system according to claim 2, further comprising
a learning section that generates the determiner by performing learning for each surgical image group that has been classified.

6. The surgery support system according to claim 5, further comprising
a supervised data generator that generates the label information.

7. The surgery support system according to claim 6, wherein the supervised data generator generates the label information by performing at least one of a hemorrhage detection to detect hemorrhage, a reworking detection to detect reworking, or a hemostasis detection to detect execution of hemostasis.

8. The surgery support system according to claim 1, further comprising
an alert controller that causes an output section to output an alert in a case where the occurrence of the dangerous condition is predicted by the predictor, the alert warning that the occurrence of the dangerous condition has been predicted.

9. The surgery support system according to claim 8, wherein
in the case where the occurrence of the dangerous condition is predicted, the predictor generates prediction information related to a prediction, and
the alert controller causes the alert to be outputted depending on the prediction information.

10. The surgery support system according to claim 9, wherein
the surgery support system includes a plurality of the output sections, and
the alert is outputted from, out of the plurality of output sections, the output section corresponding to the prediction information.

11. The surgery support system according to claim 9, wherein the prediction information includes at least one piece of information selected from a type of the dangerous condition the occurrence of which has been predicted, a degree of risk of the dangerous condition, and a position at which the dangerous condition is predicted to occur.

12. The surgery support system according to claim 1, wherein the dangerous condition includes an accidental symptom or an event that causes the accidental symptom.

13. The surgery support system according to claim 1, wherein the surgical image group includes a plurality of moving images, and the surgical image is a moving image.

14. An information processing apparatus comprising:
a storage that stores a determiner, the determiner being obtained by learning a surgical image group using, as supervised data, label information indicating a dangerous condition during surgery; and
a predictor that performs a prediction of occurrence of a dangerous condition by using a surgical image as an input and using the determiner.

15. A program causing a computer to implement
a function of storing a determiner, the determiner being obtained by learning a surgical image group using, as supervised data, label information indicating a dangerous condition during surgery, and
a function of performing a prediction of occurrence of a dangerous condition by using a surgical image as an input and using the determiner.
